# FASCICULE DE BREVET EUROPEEN

(11) **EP 1 784 236 B1**
(45) Date de publication et mention de la délivrance du brevet: **25.10.2017**
(21) Numéro de dépôt: 05767982.1
(22) Date de dépôt: 11.07.2005
(51) Int. Cl.: A61M 15/00

(54) **DISPOSITIF D'INHALATION COMPORTANT UN ENSEMBLE DE BLISTERS**
INHALATIONSVORRICHTUNG MIT EINER BLISTERPACKUNG
INHALATION DEVICE COMPRISING A BLISTER ASSEMBLY

(30) Priorité: 09.07.2004 FR 0451480
(43) Date de publication de la demande: 16.05.2007
(73) Titulaire: Aptar France SAS, 27110 Le Neubourg (FR)
(72) Inventeur: QUONIAM, Michel, F-27320 La Madeleine de Nonancourt (FR)
(74) Mandataire: CAPRI
(86) Numéro de dépôt international: PCT/EP2005/053313
(87) Numéro de publication internationale: WO 2006/010704

(56) Documents cités:
- EP-A- 0 467 172
- WO-A-01/72605
- WO-A-2004/103446
- US-A- 5 497 763
- US-A- 5 794 613

## Description

La présente invention concerne un dispositif d'inhalation comportant un ensemble de blisters.

Pour distribuer sélectivement des doses de produit fluide, notamment de poudre pharmaceutique, dans un dispositif d'inhalation, on utilise généralement des réservoirs prédosés appelés blisters. Chaque blister contient une dose de produit qui est distribuée à chaque actionnement du dispositif. Les blisters peuvent être formés sur des ensembles de blisters comportant une pluralité de blisters, et il a été déjà proposé de réaliser ces ensembles de blisters sous la forme de bande allongée ou de disque. Un problème qui se pose concerne le déplacement de l'ensemble de blisters pour amener, à chaque actionnement, un blister en position pour être ouvert afin de permettre la distribution du produit qu'il contient. La précision de ce déplacement et du positionnement de l'ensemble de blisters est primordiale pour assurer un fonctionnement sure et fiable et une précision de dosage, en particulier en fin de cycle du dispositif d'inhalation, c'est-à-dire lorsque les dernières doses sont distribuées. En particulier dans le cadre d'une bande ou ruban longitudinale, celle-ci peut être composée d'une ou plusieurs couches de matériaux relativement déformables. Pour déplacer ledit ruban, on applique sur lui un effort plus ou moins important, et le caractère déformable du ruban ou de la bande de blisters induit que ce ruban risque de se déformer au fur et à mesure que l'effort d'avancement est exercé sur lui. Cette déformation peut être préjudiciable à la précision de positionnement du ruban, notamment en fin de cycle, avec les inconvénients susmentionnés qui peuvent apparaître. Un moyen pour résoudre ce problème est de réaliser les ensembles de blisters avec des trous ou perforations au niveau des bords latéraux dudit ensemble de blisters, une ou plusieurs roues dentées venant pénétrer dans lesdites perforations pour faire avancer l'ensemble de blisters à chaque actionnement, un peu à la manière d'un film photo dans un appareil photographique. Cette mise en oeuvre implique toutefois plusieurs inconvénients. Tout d'abord, elle nécessite de réaliser l'ensemble de blisters avec une dimension en largeur augmentée afin de permettre la réalisation desdits trous. Or, plus l'ensemble de blisters est grand, plus le dispositif d'inhalation dans lequel ledit ensemble de blisters sera monté doit être également grand, ce qui peut être un inconvénient à la fois au niveau de l'encombrement, de la complexité et donc du coût de fabrication de l'assemblage. Par ailleurs, le fait de devoir réaliser des perforations dans un ensemble de blisters pouvant comporter plusieurs couches, dont certaines assez résistantes, par exemple des couches d'aluminium, pose des problèmes. Ainsi, certaines couches des ensembles de blisters multicouches sont relativement résistantes de sorte qu'au fur et à mesure que les pointes utilisées pour réaliser lesdits trous fonctionnent, elles peuvent s'éroder au contact de ces couches résistantes, ce qui peut rendre la réalisation desdits trous moins précise, et ainsi provoquer un disfonctionnement de l'inhalateur. La réalisation de tels trous latéraux implique également l'utilisation d'un système de poinçonnage complexe ce qui augmente les coûts de fabrication dudit ensemble de blisters.

Le document EP-A-0467172 divulgue une bande de blisters comportant des profils latéraux de part et d'autre de ladite bande formant une pluralité d'encoches.

Les documents WO-A-01/72605 et US-A-5 497 763 divulguent des ensembles de blisters comportant un support de blister sous forme de bande pourvue de trous ou perforations ménagé(e)s au niveau des bords latéraux. Dans un tel cas, une ou plusieurs roue(s) dentée(s) pénètre(nt) dans lesdites perforations pour faire avancer l'ensemble de blisters à chaque actionnement.

Les documents US5794613 et WO2004103446 (qui est un document selon l'article 54(3)CBE) décrivent d'autres dispositifs de l'état de la technique.

La présente invention a pour but de fournir un dispositif d'inhalation comportant un ensemble de blisters qui ne reproduit pas les inconvénients susmentionnés.

Plus particulièrement, la présente invention a pour but de fournir un dispositif d'inhalation comportant un ensemble de blisters qui soit de faible dimension et de faible encombrement.

La présente invention a également pour but de fournir un dispositif d'inhalation comportant un ensemble de blisters qui soit simple et peu coûteux à fabriquer et à assembler et fiable d'utilisation, en garantissant notamment un positionnement précis à chaque actionnement même lors des distributions des dernières doses de l'ensemble.

La présente invention a donc pour objet un dispositif d'inhalation tel que décrit dans la revendication 1. Des modes de réalisation avantageux sont exposés dans les revendications dépendantes.

D'autres caractéristiques et avantages de la présente invention apparaîtront plus clairement au cours de la description détaillée suivante de deux modes de réalisation de celle-ci faite en référence aux dessins joints, donnés à titre d'exemples non limitatifs, et sur lesquels:
- la figure 1 est une vue schématique montrant en partie supérieure un ensemble de blisters vu de dessus et en partie inférieur le même ensemble de blisters vu de côté;
- la figure 2 est une vue schématique montrant un ensemble de blisters sous forme de ruban enroulé, tel qu'il pourrait être utilisé dans un dispositif inhalation ;
- la figure 3 est une vue schématique de dessus d'une variante de réalisation d'un ensemble de blisters pour un dispositif d'inhalation selon la présente invention ; et
- les figures 4 et 5 sont des vues schématiques montrant des moyens d'entraînement coopérant avec les ensembles de blisters des figures 1 et 3 respectivement, les parties supérieures des figures 4 et 5 montrant les ensembles de blisters vus de côté, alors que les parties inférieures de figures 4 et 5 montrent ces ensembles de blisters vus de dessus.

Un ensemble de blisters pour dispositif d'inhalation comporte un support de blisters 10 pourvu d'une pluralité de blisters 11. Ce support de blisters est réalisé sous la forme d'une bande ou ruban allongé, comme représenté sur les figures. Les blisters 11 sont disposés les uns derrière les autres le long de ladite bande, avantageusement avec un écartement fixe entre eux. Chaque blister 11 du support de blisters 10 est fermé par une couche de fermeture 20. Avantageusement, cette couche de fermeture 20 peut être la même pour tout les blisters. En variante, chaque blister pourrait avoir sa propre couche de fermeture respective. Cette couche de fermeture 20 peut être ouverte lors de l'actionnement par tous moyens appropriés, par exemple par décollage, découpage, déchirage, poinçonnage, perçage ou similaire. L'ouverture des blisters ne faisant pas partie de la présente invention, ces moyens d'ouverture ne seront donc pas décrits ci-après. Cet ensemble de blisters est destiné à être monté dans un dispositif d'inhalation, et celui-ci peut être quelconque, de sorte qu'il n'est pas non plus représenté ou décrit plus en détail.

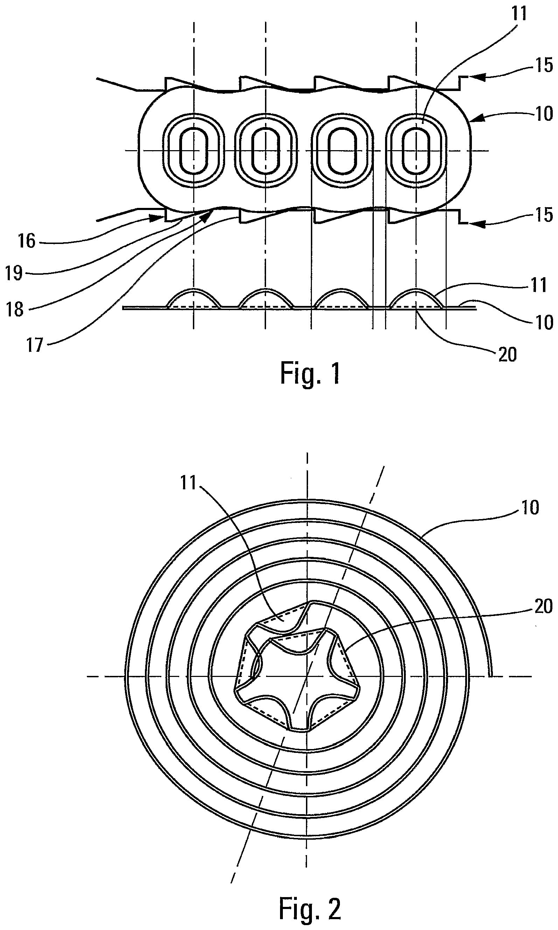

La figure 2 représente un mode de réalisation particulier, dans lequel l'ensemble de blisters est réalisé sous la forme d'une bande souple pouvant être enroulée pour être montée dans un dispositif d'inhalation, ladite bande étant ensuite progressivement déroulée à chaque actionnement du dispositif d'inhalation. D'autres variantes sont également envisageables.

Selon l'invention, le support de blisters 10 comporte deux profils latéraux 15 s'étendant le long dudit support de blisters 10. Dans le cadre d'une bande allongée, le support de blister 10 comporte deux profils latéraux 15, un de chaque côté longitudinal de ladite bande. Comme visible sur les figures, chaque profil latéral 15 comporte plusieurs projections 16 qui sont destinées à permettre un déplacement précis dudit ensemble de blisters à chaque actionnement du dispositif d'inhalation dans lequel celui-ci est monté.

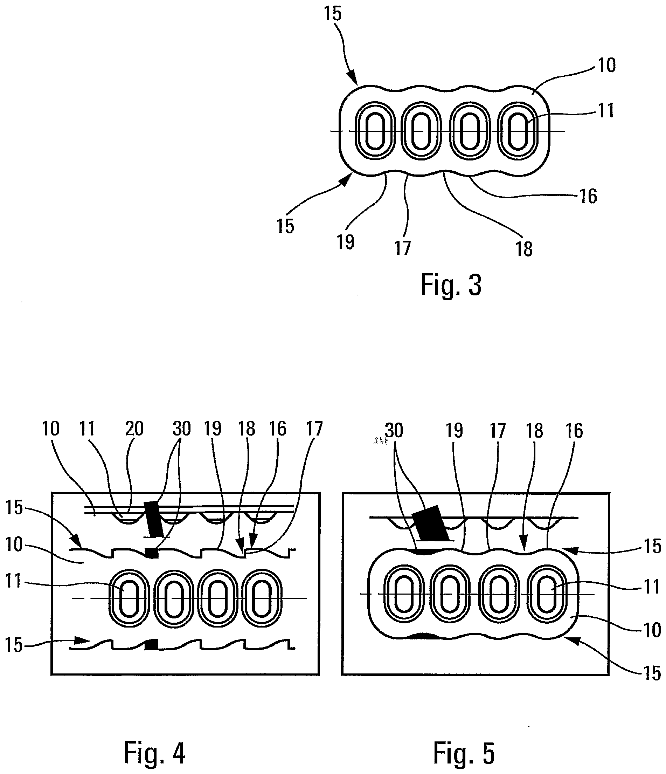

En se référant plus particulièrement à la figure 1, qui montre un premier mode de réalisation avantageux de l'invention, on constate qu'une projection 16 peut être associée à chaque blister 11 sur chaque profil latéral 15. Dans la forme de réalisation représentée, chaque blister 11 est donc associé à deux projections 16 disposées de chaque côté latéral dudit blister. Chaque profil latéral 15 est formé d'une alternance de projections 16 et de creux 18, et une surface de butée 17 est prévue au niveau de chaque projection 16. Cette surface de butée 17 est destinée à coopérer avec des moyens d'entraînement 30 du dispositif d'inhalation qui seront décrits plus précisément en référence aux figures 4 et 5. Chaque surface de butée 17 est reliée à la surface de butée 17 directement adjacente par une surface de glissement 19 permettant un déplacement régulier des moyens d'entraînement entre deux blisters, pour faciliter leurs positionnement dans la projection suivante, après chaque actionnement. Avantageusement, la surface de butée 17 est formée par une paroi qui est environ transversale par rapport à la direction long de laquelle les blisters 11 s'étendent sur ledit support de blisters 10.

Dans l'exemple particulier de la figure 1, dans lequel le support de blisters 10 est une bande allongée, la surface de butée 17 est environ perpendiculaire aux bords longitudinaux de ladite bande. Cette mise en oeuvre permet de positionner des moyens d'entraînement appropriés 30 au niveau de chaque creux 18 en contact avec une surface de butée 17 respective de la projection respective 16 pour réaliser une avancée du support de blisters 10 sans déformation notable dudit support. La présente invention permet donc un meilleur positionnement du support de blisters réalisé sous forme de bande ou de ruban en utilisant un profil qui réduit les déformations, notamment en fin de cycle d'avancement, tout en minimisant la largeur du ruban.

Les figures 3 et 5 représentent une variante de réalisation de l'invention. Cette variante de réalisation comporte également un profil latéral 15 sur chaque côté du ruban, celui-ci étant formé par des ondulations enchaînant en alternance des creux 18 et des projections 16. La surface de butée dans ce second mode de réalisation n'est pas aussi marqué que dans le premier mode de réalisation et elle n'est en particulier pas perpendiculaire au bord latéral du ruban, mais la présence de ce profil en ondulation permet également de réaliser un avancement du support de blisters sans déformation excessive dudit ruban.

Les profils latéraux représentés sur les figures 1 et 3 sont simples à fabriquer, par simple découpe des bords latéraux des rubans. On limite ainsi la largeur des supports de blisters, ce qui à un impact sur les dimensions globales de l'ensemble de blisters et par conséquent aussi du dispositif d'inhalation dans lequel il sera assemblé, notamment si l'ensemble de blisters contient un grand nombre de doses comme cela est souvent le cas.

D'autres modifications et variantes sont aussi envisageables pour l'homme du métier sans sortir du cadre de la présente invention telle que définie par les revendications annexées.

## Revendications

1. Dispositif d'inhalation comportant un ensemble de blisters et des moyens d'entraînement (30), ledit ensemble de blisters comportant un support de blisters (10) comportant une pluralité de blisters (11), chaque blister (11) étant fermé hermétiquement par une couche de fermeture (20), le support de blisters (10) étant en forme de bande allongée, les blisters (11) étant disposés les uns après les autres le long de ladite bande de blisters, ledit support de blisters (10) comportant deux profils latéraux (15), un de chaque côté longitudinal dudit support de blisters (10), lesdits profils latéraux sont formés d'une alternance de creux (18) et de projections (16), chaque projection (16) comportant une surface de butée (17), lesdits moyens d'entraînement (30) étant adaptés à coopérer à chaque actionnement avec une surface de butée (17) respective d'une projection (16) respective de chaque profil latéral (15) pour déplacer d'une manière précise ledit ensemble de blisters à chaque actionnement dudit dispositif d'inhalation, **caractérisé en ce que** chaque surface de butée (17) est reliée à la surface de butée adjacente (17) par une surface de glissement (19) formée par chaque creux, ladite surface de glissement (19) permettant un déplacement régulier desdits moyens d'entraînement (30) entre deux blisters, pour faciliter leur positionnement dans la projection suivante après chaque actionnement.

2. Dispositif selon la revendication 1, dans lequel ladite surface de butée (17) est formée par une paroi (17) environ transversale par rapport à la direction le long de laquelle les blisters (11) s'étendent sur ledit support de blisters (10).

3. Dispositif selon la revendication 1 ou 2, dans lequel chaque surface de butée (17) est environ perpendiculaire aux bords longitudinaux dudit support de blisters (10) en forme de bande.

4. Dispositif selon l'une que quelconque des revendications précédentes, dans lequel ledit support de blisters (10) est une bande souple allongée apte à être enroulée pour être montée dans un dispositif d'inhalation, et à se dérouler progressivement à chaque actionnement.

5. Dispositif selon l'une quelconque des revendications précédentes, dans lequel chaque blister (11) est formé par une cavité, notamment une cavité rigide, renfermant une dose de poudre à distribuer par ledit dispositif d'inhalation.

## Patentansprüche

1. Inhalationsvorrichtung, aufweisend einen Blistersatz und Mitnahmemittel (30), wobei der Blistersatz einen Blisterträger (10) aufweist, der mehrere Blister (11) aufweist, wobei jeder Blister (11) durch eine Verschlussschicht (20) hermetisch verschlossen ist, wobei der Blisterträger (10) in Form eines langgestreckten Streifens vorliegt, wobei die Blister (11) entlang des Blisterstreifens nacheinander angeordnet sind, wobei der Blisterträger (10) zwei Seitenprofile (15) aufweist, und zwar eines an jeder Längsseite des Blisterträgers (10), wobei die Seitenprofile abwechselnd aus Vertiefungen (18) und Vorsprüngen (16) gebildet sind, wobei jeder Vorsprung (16) eine Anschlagfläche (17) aufweist, wobei die Mitnahmemittel (30) dazu geeignet sind, bei jeder Betätigung mit einer Anschlagfläche (17) jeweils eines Vorsprungs (16) jeweils eines jeden Seitenprofils (15) zusammenzuwirken, um den Blistersatz bei jeder Betätigung der Inhalationsvorrichtung präzise zu verschieben, **dadurch gekennzeichnet, dass** jede Anschlagfläche (17) mit der angrenzenden Anschlagfläche (17) durch eine Gleitfläche (19) verbunden ist, die durch jede Vertiefung gebildet ist, wobei die Gleitfläche (19) eine gleichmäßige Verschiebung der Mitnahmemittel (30) zwischen zwei Blistern ermöglicht, um nach jeder Betätigung ihre Positionierung in dem nachfolgenden Vorsprung zu vereinfachen.

2. Vorrichtung nach Anspruch 1, wobei die Anschlagfläche (17) durch eine Wand (17) gebildet ist, die in Bezug auf die Richtung, entlang welcher sich die Blister (11) auf dem Blisterträger (10) erstrecken, quer steht.

3. Vorrichtung nach Anspruch 1 oder 2, wobei jede Anschlagfläche (17) zu den Längsrändern des Blisterträgers (10) in Streifenform in etwa senkrecht steht.

4. Vorrichtung nach einem der vorhergehenden Ansprüche, wobei der Blisterträger (10) ein flexibler, langgestreckter Streifen ist, der aufgerollt werden kann, um in einer Inhalationsvorrichtung montiert zu werden, und der bei jeder Betätigung nach und nach abgerollt werden kann.

5. Vorrichtung nach einem der vorstehenden Ansprüche, wobei jeder Blister (11) durch einen Hohlraum, insbesondere einen starren Hohlraum, gebildet ist, der eine Pulverdosis zur Ausgabe durch die Inhalationsvorrichtung enthält.

## Claims

1. An inhaler including a blister pack and drive means (30), said blister pack comprising a blister support (10) with a plurality of blisters (11), each blister (11) being hermetically sealed by a closure layer (20), the blister support (10) being in the form of an elongate strip, the blisters (11) being disposed one after the other along said blister support strip, said blister support (10) including two lateral profiles (15), one on either longitudinal side of said blister support (10), said lateral profiles being formed by alternating depressions (18) and projections (16), each projection (16) including an abutment surface (17), said drive means (30) being adapted to co-operate on each actuation with a respective abutment surface (17) of a respective projection (16) of each lateral profile (15), for displacing said blister pack in accurate manner each time said inhaler is actuated, the blister pack being **characterized in that** each abutment surface (17) is connected to the adjacent abutment surface (17) via a slide surface (19) formed by each depression, said slide surface (19) allowing a smooth displacement of said drive means (30) between two blisters, to facilitate their positioning in the next projection after each activation.

2. An inhaler according to claim 1, in which said abutment surface (17) is formed by a wall (17) that is approximately transverse relative to the direction along which the blisters (11) extend over said blister support (10).

3. An inhaler according to claim 1 or claim 2, in which each abutment surface (17) is approximately perpendicular to the longitudinally-extending edges of said strip-shaped blister support (10).

4. An inhaler according to any preceding claim, in which said blister support (10) is an elongate flexible strip that is suitable for being rolled up so as to be mounted in an inhaler.

5. An inhaler according to any preceding claim, in which each blister (11) is formed by a cavity, in particular a rigid cavity, containing one dose of powder to be dispensed by said inhaler.
